# EUROPEAN PATENT APPLICATION

(11) **EP 2 518 167 A1**
(43) Date of publication of application: **31.10.2012**
(21) Application number: 11164279.9
(22) Date of filing: 29.04.2011
(51) Int. Cl.: C12R 1/01

(54) **Bacteria for second generation bioethanol production**

(71) Applicant: Technical University of Denmark, 2800 Kgs. Lyngby (DK)
(72) Inventor: Angelidaki, Irini, 2000 Frederiksberg (DK); Tomas, Ana F., 2200 Copenhagen N (DK); Karakashev, Dimitar Borisov, 2860 Buddinge (DK)
(74) Representative: Høiberg A/S

(57) **Abstract**

The present invention relates to a novel anaerobic, extreme thermophilic, ethanol high-yielding bacterium. The invention is based on the isolation of the bacterial strain referred to herein as "DTU01", which produces ethanol as the main fermentation product, followed by acetate and lactate. The isolated organism is an extremely interesting and very promising organism for the establishment of a sustainable bioethanol production process. The invention further relates to a method for producing a fermentation product such as ethanol.

## Description

### Field of invention

The present invention relates to a novel anaerobic, extreme thermophilic, ethanol high-yielding bacterium.

### Background of invention

A renewed interest in alternative sources of energy has been rising in the last decade, together with the awareness that the use of fossil fuels has been causing great damage to the environment. The depletion of the planet's natural resources has led to increased efforts of establishing processes for alternative fuel production that comply with the definitions of sustainability and environmental-friendly.

The production of bioethanol from biomass-derived sugars referred to as 'first-generation' bioethanol is now an established and mature process. In 2008, of a total of 65 billion litres of ethanol produced worldwide, 89 % were produced in the USA and Brazil. However, the main feedstock for ethanol production in the USA has been the processed starch fraction of yellow corn (maize), while in Brazil sucrose from sugar cane is used. The use of land resources to produce biofuels instead of food or food grade resources has raised criticism and therefore alternative feedstocks need to be used, so that bioethanol production becomes truly sustainable.

Agricultural residues such as straw, corn stover, bagasse, and waste such as household waste, also contain fermentable carbohydrates and can be used for ethanol production in the so called 'second-generation' processes. However, often the agricultural organic residues have lignocellulosic structure, and therefore, the carbohydrates are not readily available to the traditionally used fermenting microorganisms. Moreover, following efficient pre-treatment, lignocellulosic biomass can yield more than 20% of pentose sugars, such as xylose and arabinose, which cannot be used by native strains of *Saccharomyces cerevisae,* the most used organism in first-generation bioethanol production.

Thus, in contrast to starch, the hydrolysis of lignocellulosic biomass results in the release of pentose sugars in addition to hexose sugars. This implies that useful fermenting organisms need to be able to convert both hexose and pentose sugars to a desired fermentation product such as ethanol.

Gram-positive thermophilic bacteria have unique advantages over the conventional ethanol production strains like *Sacchararomyces cerevisiae* and *Zymomonas mobilis.* The primary advantages are their broad substrate specificities and high natural ability for production of ethanol. Moreover, ethanol fermentation at high temperatures (55-80° C) has many advantages over mesophilic fermentation. One advantage of thermophilic fermentation is the minimisation of the problem of contamination in continuous cultures, since only a few microorganisms are able to grow at such high temperatures in undetoxified lignocellulosic hydrolysate.

Presently, dependent on the pre-treatment method, cellulases and hemicellulases often have to be added to the pre-treated lignocellulosic hydrolysate in order to release sugar- monomers. These enzymes contribute significantly to the production costs of the fermentation products. However, many thermophilic gram-positive strains possess a range of the relevant enzymes and supplementary additions could become less expensive if a thermophilic gram-positive strain is used. Fermentation at high temperature also has the additional advantages of high productivities and substrate conversions and facilitated product recovery.

Lignocellulose hydrolysates contain inhibitors such as furfural, phenols and carboxylic acids, which can potentially inhibit the fermenting organism. Therefore, the organism must also be tolerant to these inhibitors. The inhibitory effect of the hydrolysates can be reduced by applying a detoxification process prior to fermentation. However, the inclusion of this extra process step increases significantly the total cost of the fermentation product and should preferably be avoided. It is therefore preferred that the microorganism is capable of producing fermentation products from undetoxified hemicellulose or holocellulose hydrolysates to make it usable in an industrial lignocellulosic-based fermentation process due to the high cost of detoxification process.

It is also particularly advantageous if the potential microorganism is capable of growing on high concentrations of lignocellulosic hydrolysates, i.e. lignocellulosic hydrolysates with high dry-matter content. This is of particular importance when the microorganism is for alcohol production such as ethanol production, since distillation costs increase with decreasing concentrations of alcohol.

Engineered strains of *Thermoanaerobacter mathranii* (WO 2007/134607, Georgieva & Ahring, 2007), *Geobacillus thermoglucosidasius* (Cripps et al., 2009) and *Thermoanaerobacterium saccharolyticum* (Shaw et al., 2009) can produce up to 0.44 g ethanol g⁻¹ sugar consumed, corresponding to 86% of the theoretical yield (0.51 g ethanol g⁻¹ sugar consumed for glucose and xylose). However, these organisms have yet to achieve the high yields and ethanol tolerance that are characteristic of the commonly used mesophilic species, such as *Saccharomyces cerevisae* or *Zymomonas mobilis***.** The latter can typically achieve active growth in up to 12-14 % w/w ethanol and produce up to 0.50 g ethanol g⁻¹ sugar consumed, i.e. almost 100% of the theoretical yield.

### Summary of invention

The present invention relates to a novel anaerobic, extreme thermophilic, ethanol high-yielding bacterium (DSMZ Accession number 24726) isolated from household waste and mutants thereof. The invention is based on the isolation of the bacterial strain referred to herein as "DTU01", which produces ethanol as the main fermentation product, followed by acetate and lactate.

The non-genetically modified DTU01 strain according to the present invention can produce up to 1.28 mol_{ethanol/}mol_{xylose} consumed (0.39 g ethanol g⁻¹ sugar consumed) corresponding to 77% of the theoretical yield. This is so far the highest yielding ethanol producing organism from pentoses, which is not genetically modified.

The isolated organism is therefore an extremely interesting and very promising organism for the establishment of a sustainable ethanol production process.

### Definitions

Bioethanol: Ethanol derived from plant materials.

Caldicoprobacter DTU01: or simply "DTU01" refers to the bacterial strain deposited under DSMZ Accession number 24726 on 8 April 2011.

CSTR: continuously stirred tank reactor.

Furfural: Furfural is an organic compound derived from a variety of agricultural byproducts, including corncobs, oat, wheat bran, and sawdust. Furfural within lignocellulosic materials can potentially inhibit the fermenting organism.

Hydraulic retention time: The Hydraulic retention time (HRT) also known as Hydraulic residence time or t (tau), is a measure of the average length of time that a soluble compound remains in a constructed bioreactor.

Lignocellulosic hydrolysate: In the present context the term "lignocellulosic hydrolysate" is intended to designate a lignocellulosic biomass which has been subjected to a pre-treatment step whereby lignocellulosic material has been at least partially separated into cellulose, hemicellulose and lignin thereby having increased the surface area of the material. The lignocellulosic material may typically be derived from plant material, such as straw, hay, garden refuse, comminuted wood, fruit hulls and seed hulls.

Mesophilic microorganism: A microorganism that grows at moderate temperatures, i.e. neither too hot nor too cold, typically between 25 and 40 °C

Microorganism: In the present context the term "microorganism" is used interchangeably with the term "bacterium".

OD: optical density.

Theoretical yield: The theoretical yield of a reaction is the amount of product that would be formed if the reaction went to completion. It is based on the stoichiometry of the reaction and ideal conditions in which starting material is completely consumed, undesired side reactions do not occur, the reverse reaction does not occur, and there no losses in the work-up procedure. The theoretical yield of ethanol is 0.51 g ethanol g-¹ sugar consumed for glucose and xylose.

Thermophilic microorganism: A thermophilic microorganism grows at relatively high temperatures, typically between 45 and 80 °C.

VFA: volatile fatty acid.

Xylose: Xylose is a pentose. Xylose is the main building block for hemicellulose, which comprises about 30% of plant matter.

YE: Yeast extract.

### Detailed description of the invention

The present invention relates to a new extreme thermophilic microorganism and mutants thereof.

The invention is based on the isolated thermophilic bacterium, herein referred to as "DTU01". The bacterium has been deposited in accordance with the terms of the Budapest Treaty on 8 April 2011 with DSMZ - Deutsche Sammlung von Mikroorganismen und Zellkulturen GmbH, Inhoffenstraße 7 B, 38124 Braunschweig, Germany under DSMZ accession number 24726.

The deposited bacterium has not been genetically modified. Other bacteria of the present invention can therefore be obtained by mutating the deposited bacteria and selecting derived mutants having enhanced characteristics. Desirable characteristics include an increased range of sugars that can be utilised, increased growth rate, ability to produce higher amounts of fermentation products such as ethanol, increased tolerance to ethanol, increased tolerance to other inhibitors such as furfural, phenols and carboxylic acids etc. Suitable methods for mutating bacteria and selecting desired mutants are described in e.g. Functional analysis of Bacterial genes: A practical Manual, edited by W. Schumann, S. D. Ehrlich & N. Ogasawara, 2001.

The microorganism of the present invention is capable of growing and producing fermentation products of lignocellulosic hydrolysates. In the present context the term "lignocellulosic hydrolysate" is intended to designate a lignocellulosic biomass which has been subjected to a pre-treatment step whereby lignocellulosic material has been at least partially separated into cellulose, hemicellulose and lignin thereby having increased the surface area of the material. The lignocellulosic material may typically be derived from plant material, such as straw, hay, garden refuse, comminuted wood, fruit hulls and seed hulls.

The pre-treatment method most often used is acid hydrolysis, where the lignocellulosic material is subjected to an acid such as sulphuric acid whereby the sugar polymers cellulose and hemicellulose are partly or completely hydrolysed to their constituent sugar monomers. Another type of lignocellulose hydrolysis is steam explosion, a process comprising heating of the lignocellulosic material by steam injection to a temperature of 190-230°C. A third method is wet oxidation wherein the material is treated with oxygen at 150-185°C. The pre- treatments can be followed by enzymatic hydrolysis to complete the release of sugar monomers. This hydrolytic step results in the hydrolysis of cellulose into glucose while hemicellulose is transformed into the pentoses xylose and arabinose and the hexoses glucose, galactose and mannose. The pre-treatment step may in certain embodiments be supplemented with additional treatment resulting in further hydrolysis of the cellulose and hemicellulose. The purpose of such an additional hydrolysis treatment is to hydrolyse oligosaccharide and possibly polysaccharide species produced during the acid hydrolysis, wet oxidation, or steam explosion of cellulose and/or hemicellulose origin to form fermentable sugars (e.g. glucose, xylose and possibly other monosaccharides). Such further treatments may be either chemical or enzymatic. Chemical hydrolysis is typically achieved by treatment with an acid, such as treatment with aqueous sulphuric acid, at a temperature in the range of about 100-150°C. Enzymatic hydrolysis is typically performed by treatment with one or more appropriate carbohydrase enzymes such as cellulases, glucosidases and hemicellulases including xylanases.

The microorganism of the present invention is capable of growing in a medium comprising a hydrolysed lignocellulosic biomass material having a dry-matter content of at least 5% w/v, including 10% w/v, such as at least 15% w/v, including at least 20% w/v, and even as high as at least 25% wt/wt. As mentioned previously, this has the great advantage that it may not be necessary to dilute the hydrolysate before the fermentation process, and thereby it is possible to obtain higher concentrations of fermentation products such as ethanol, and thereby the costs for subsequently recovering the fermentation products may be decreased. For example the distillation costs for ethanol will increase with decreasing concentrations of alcohol.

The microorganism according to the invention is an anaerobic thermophilic bacterium, which is capable of growing at high temperatures such as at or even above 70°C. The fact that the strain is capable of operating at this high temperature is of high importance in the conversion of the lignocellulosic material into fermentation products. The conversion rate of carbohydrates into e.g. ethanol is much faster when conducted at high temperatures. For example, ethanol productivity in a thermophilic Bacillus is up to ten-fold faster than a conventional yeast fermentation process which operates at 30°C. Consequently, a smaller production plant is required for a given volumetric productivity, thereby reducing plant construction costs. As also mentioned previously, at high temperature, there is a reduced risk of contamination from other microorganisms, resulting in less downtime, increased plant productivity and a lower energy requirement for feedstock sterilisation. The high operation temperature may also facilitate the subsequent recovery of the resulting fermentation products.

The microorganism of the present invention may be used for second generation bioethanol production from lignocellulosic materials. Lignocellulosic materials or biomass refers to plant biomass that is composed of cellulose, hemicellulose, and lignin. The carbohydrate polymers (cellulose and hemicelluloses) are tightly bound to the lignin. Lignocellulosic biomass can be grouped into four main categories: agricultural residues (including corn stover and sugarcane bagasse), dedicated energy crops, wood residues (including sawmill and paper mill discards), and municipal paper waste. Lignocellulosic materials according to the present invention may typically be derived from plant material, such as straw, hay, garden refuse, comminuted wood, fruit hulls and seed hulls. These materials should not however be construed as limiting for the present invention.

Numerous fermentation products are valuable commodities which are utilised in various technological areas, including the food industry and the chemical industry. Presently, the increasing global energy requirements have resulted in increasing focus on alternatives to fossil fuels as energy sources, and ethanol derived from plant materials (bioethanol) has received particular attention as a potential replacement for or supplement to petroleum-derived liquid hydrocarbon products.

Lactic acid, which is another fermentation product, is extensively used in the cosmetics industry as an anti-aging chemical, and the food industry use lactic acid in a variety of food stuffs to act as an acidity regulator. Recently, lactic acid has also attracted much attention for its potential use in biodegradable polyesters.

The strain according to invention has the potential to be capable of producing a number of different fermentation products, including but not limited to ethanol, acetate, lactate, hydrogen, propanol and propionate.

In one embodiment, the microorganism of the present invention is capable of producing bioethanol with high yield compared to other thermophilic bacteria.

In one embodiment, the microorganism of the present invention produces ethanol as the main fermentation product.

The microorganism of the present invention is able to grow at temperatures ranging from 50 to 80°C, such as 55 to 75°C, including 60 to 75°, such as 65 to 75°C. The temperature optimum of the non-modified microorganism was determined to be 70°C.

The microorganism of the present invention is able to grow at a pH ranging from 4.5 to 9, such as 5 to 8.5, including 5.5 to 8, such as 6 to 8, including 6.5 to 7.5. The pH optimum of the non-modified microorganism was determined to be pH 7.

Depending on the type of downstream processing, a low ethanol tolerance can be a major obstacle for the commercial exploitation of thermophilic bacteria for bioethanol production and selection for ethanol resistant strains is therefore of great importance. However, if ethanol is continuously removed during production, a high ethanol tolerance is not necessary. Non-modified DTU01 is able to grow unaffected in up to 2% v/v ethanol and it can still grow, albeit at slower rates, at 4% v/v ethanol in the medium. Adaptation to higher concentrations of ethanol can be achieved by repeated batch cultivation of the microorganism to obtain strains that are more tolerant to ethanol. However, if in the industrial process ethanol is to be evaporated (due to the thermophilic temperature) and removed continuously, it may not be necessary to improve the ethanol tolerance of DTU01 at all.

The microorganism of the present invention is capable of utilizing the following substrates: Arabinose, Cellobiose, Cellulose, Fructose, Galactose, Glucose, Lactose, Mannitol, Mannose, Melibiose, Starch, Sucrose, Xylan, Xylose.

In one embodiment, the microorganism of the present invention is capable of converting pentoses, such xylose to ethanol with high yield.

In one embodiment, the microorganism of the present invention is capable of growing on and producing fermentation products from concentrations of pentoses ranging from 2 to 60 g/I, such as 5 to 55 g/I, for example 10 to 55 g/I, such as 15 to 55 g/I, for example 20 to 55 g/I, such as 25 to 55 g/I, for example 30 to 55 g/I, such as 35 to 55 g/I, for example 40 to 55 g/I, such as 45 to 55 g/I.

In one embodiment, the microorganism of the present invention is capable of growing on and producing fermentation products from concentrations of pentoses ranging from 2 to 60 g/I, such as 2 to 55 g/I, for example 2 to 50 g/I, such as 2 to 45 g/I, for example 2 to 40 g/I, such as 2 to 35 g/I, for example 2 to 30 g/I, such as 2 to 25 g/I, for example 2 to 20 g/I.

In one embodiment, the microorganism of the present invention is capable of growing on and producing fermentation products from concentrations of pentoses ranging from 2 to 60 g/I, such as 5 to 55 g/I, for example 10 to 50 g/I, such as 15 to 45 g/I, for example 20 to 40 g/I, such as 25 to 35 g/I.

In one embodiment, the microorganism of the present invention is capable of converting hexoses, such glucose, to ethanol with high yield.

As opposed to starchy or cellulosic substrates, which are almost exclusively broken down to glucose, lignocellulosic biomass contains several different sugar monomers, including both hexoses and pentoses. If these are to be converted into a fermentation product such as ethanol in a continuous process, it is necessary for all the sugars to be taken up and metabolized simultaneously (co-fermentation).

The microorganism of the present invention is capable of co-fermenting hexoses and pentoses.

The microorganism of the present invention is able to grow and produce significant yields of ethanol in media lacking nutrient sources such as vitamins, peptone or YE. This is a valuable asset for a candidate microorganism for second-generation bioethanol production, meaning this can be achieved without costly addition of supplements to the lignocellulosic biomass.

The hydrolysis of lignocellulosic biomass results in the release of microbial inhibitors, hence in a preferred embodiment, the microorganism of the present invention exhibits tolerance to substances that may inhibit the growth and fermentation of the microorganism. Substances that may inhibit the growth and fermentation of the microorganism include, but are not limited to ethanol, high levels of substrates (substrate inhibition), furfural, phenols and carboxylic acids. Tolerance to said inhibitors can be improved by culturing the microorganism in increasing concentrations of the inhibitors.

DTU01 may in advantageous embodiments be modified in order to obtain mutants or derivatives thereof, with improved characteristics. Thus, in one embodiment there is provided a bacterial strain according to the invention which is a variant or mutant of DTU01 wherein one or more genes have been inserted, deleted or substantially inactivated. Genes may be inserted, deleted or substantially inactivated using suitable gene manipulation tools and genetic engineering procedures which are well known in the art, e.g. gene cloning systems, homologous recombination and techniques described in Sambrook & Russell "Molecular Cloning: A Laboratory Manual" (Third Edition), Cold Spring Harbor Laboratory Press.

As mentioned above, DTU01 possesses the genetic machinery to enable it to convert both hexose sugars and pentose sugars to a range of desired fermentation products, including ethanol. However, it may for certain embodiments be desired to insert one or more additional genes into the bacterium according to the invention. Thus, in order to improve the yield of a particular fermentation product, it may be beneficial to insert one or more genes encoding a polysaccharase into the bacterium according to the invention. Hence, in specific embodiments there is provided a bacterium according to the invention wherein one or more genes encoding a polysaccharase which is selected from cellulases (such as EC 3.2.1.4); beta-glucanases, including glucan-1,3 beta-glucosidases (exo-1,3 beta-glucanases, such as EC 3.2.1.58), 1,4- beta-cellobiohydrolase (such as EC 3.2.1.91) and endo-I,3(4)-beta-glucanases (such as EC 3.2.1.6); xylanases, including endo-I,4-beta-xylanases (such as EC 3.2.1.8) and xylan 1,4- beta-xylosidase (such as EC 3.2.1.37); pectinases (such as EC 3.2.1.15); alpha-glucuronidase, alpha-L-arabinofuranosidase (such as EC 3.2.1.55), acetylesterase (such as EC 3.1.1.-), acetylxylanesterase (such as EC 3.1.1.72), alpha amylase (such as EC 3.2.1.1), beta-amylase (such as EC 3.2.1.2), glucoamylase (such as EC 3.2.1.3), pullulanase (such as EC 3.2.1.41), beta-glucanase (such as EC 3.2.1.73), hemicellulase , arabinosidase, mannanases including mannan endo-I,4-beta-mannosidase (such as EC 3.2.1.78) and mannan endo-I,6-alpha-mannosidase (such as EC 3.2.1.101), pectin hydrolase, polygalacturonase (such as EC 3.2.1.15), exopolygalacturonase (such as EC 3.2.1.67) and pectate lyase (such as EC 4.2.2.10) have been inserted in the bacterium.

Depending on the desired fermentation product, it is contemplated that in certain embodiments it is useful to insert heterologous genes encoding a pyruvate decarboxylase (such as EC 4.1.1.1) or to insert a heterologous gene encoding an alcohol dehydrogenase (such as EC 1.1.1.1, EC 1.1.1.2, EC 1.1.1.71, or EC 1.1.99.8) or to up-regulate an already existing gene encoding alcohol dehydrogenase, acetaldehyde dehydrogenase, pyruvate decarboxylase, pyruvate dehydrogenase.

It is further contemplated to knock-out genes that lead to the formation of un-wanted secondary products, such lactate dehydrogenase, acetate kinase, pyruvate formate lyase, phosphate acetyltransferase.

In accordance with the invention a method of producing a fermentation product comprising culturing a microorganism according to the invention under suitable conditions is also provided.

The bacterium according to the invention is a strict anaerobic microorganism, and hence it is preferred that the fermentation product is produced by a fermentation process performed under strict anaerobic conditions. Additionally, the strain according to the invention is a thermophillic microorganism, and therefore the process may perform optimally when it is operated at temperatures in the range of about 40-95°C, such as the range of about 50-90°C, including the range of about 60-85°C, such as the range of about 65-75°C. In a preferred embodiment, the fermentation process is operated at or about 70°C.

For the production of certain fermentation products, it may be useful to select a specific fermentation process, such as batch fermentation process, including a fed batch process or a continuous fermentation process. In accordance with the invention, the method is useful for the production of a wide range of fermentation products. Thus fermentation products such as ethanol, acetate, lactate, hydrogen, propanol and propionate may be produced in accordance with the invention. A preferred fermentation product according to the invention is ethanol.

The invention will now be further described in the following non-limiting examples and figures.

### Detailed description of Figures

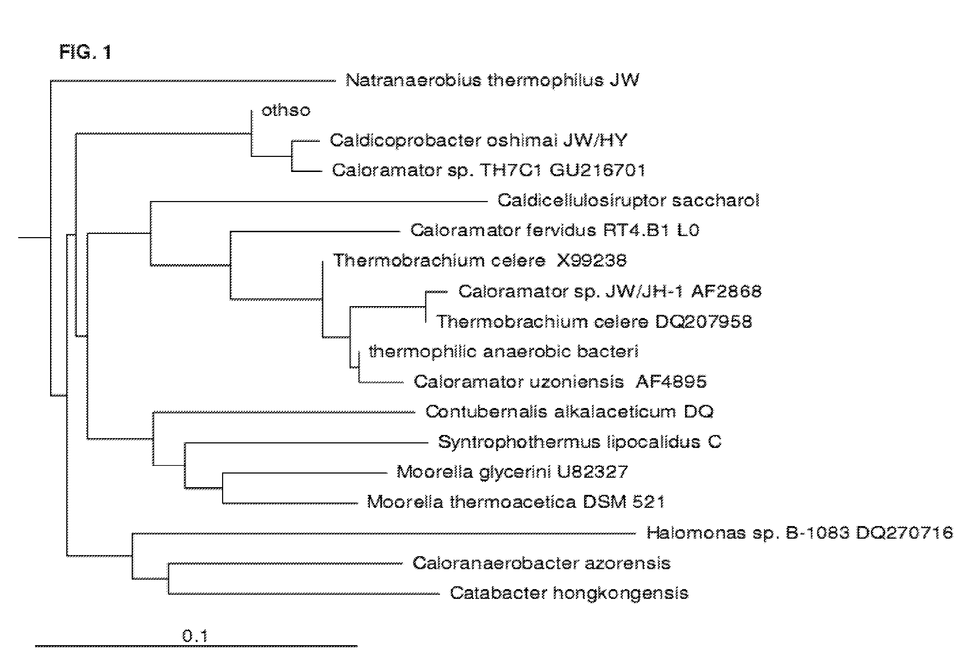

**Figure 1**. Phylogenetic position of strain DTU01 (referred to as "othso" in the figure) within the *Clostridium* subphylum of Gram-positive bacteria. A total of 1302 sites were used for the phylogenetic analysis. Alignment was performed with representatives of cluster XII, with representatives of cluster XIII as an outgroup. The topology shown is an unrooted tree obtained by a neighbourjoining algorithm (Jukes & Cantor corrections) established using PHYLOjWIN and performed manually using SEAVIEW. Significant bootstrap values (calculated from 500 trees) are indicated as percentages at the branching points. Bar, 5±9 nt substitutions per 100 nt.
**Figure 2****.** Time course of ethanol production and xylose consumption for different initial concentrations of xylose.
**Figure 3****.** Ethanol yield, ethanol to acetate and lactate ratio, and xylose removal percentages for different initial concentrations of xylose.
**Figure 4****.** Xylose consumption, ethanol yield and final concentration of fermentation products (ethanol, acetate, lactate) as a function of different initial concentrations of YE and peptone.
**Figure 5****.** Time course of ethanol production and xylose consumption for different medium compositions. The tests where vitamins and YE were omitted from the medium were performed at pH 7. : No V - medium lacking vitamin solution; No V, no YE - medium lacking vitamin solution and YE. pH 7 & pH 5.5 - media supplemented with 1 g/I YE and 1 ml/I vitamin solution.
**Figure 6****.** Representation of ethanol yield, percentage of xylose consumed and ethanol to acetate and lactate ratio for the different medium compositions tested: No V - medium lacking vitamin solution; No V, no YE - medium lacking vitamin solution and YE. pH 7 & pH 5.5 - media supplemented with 1 g/I YE and 1 ml/I vitamin solution.
**Figure 7****.** Photomicrographs of vegetative cells (top) and sporulating cells (bottom) of strain DTU01.

### Examples

### MATERIALS AND METHODS

### Inoculum and isolation

The inoculum was a high-yielding ethanol and hydrogen mixed culture (Zhao et al., 2009) enriched from a bio-hydrogen producing reactor operation at 70°C using xylose as the main carbon source. The initial inoculum was obtained from a laboratory scale bio-hydrogen producing continuously stirred tank reactor (CSTR), fed with household solid waste. This reactor was operated at 70°C and had a hydraulic retention time of 3 days.

The mixed culture had previously achieved 95 % of the theoretical ethanol yield from the pentose, xylose under specific cultivation conditions (Zhao et al., 2010), which included the supply of high concentrations of nutritional supplements and nitrogen sources such as yeast extract (YE) and peptone, which would be undesirable in a large-scale process. Dominant organisms in this enrichment culture were phylogenetically affiliated to some representatives of the genus *Thermoanaerobacter*. Although mixed cultures are also able to produce high yields of bioethanol, ultimately a single strain is desirable, as relatively small changes of the environmental conditions, could give advantage to other species in the mixed culture, with different fermentation product profiles as a result.

Isolation was carried out following the roll-tube technique as previously described. Series of dilutions of the enrichment cultures were transferred into a modified basic anaerobic (BA) medium: cysteine hydrochloride was omitted, 1 g yeast extract I⁻¹ was added, and it was supplied with 11 g Gelrite I⁻¹ and 2 g MgCl₂I⁻¹ to ensure that the medium remained solid at the incubation temperature of 70 °C. 5 g xylose I⁻¹ was used as the carbon source throughout the experiment, except where noted. Colonies were picked with sterile Pasteur pipettes, diluted and transferred to new roll tubes. This procedure was repeated until only one colony type was present and 3 times more to ensure purity.

### Medium and batch cultivations

The cultivation medium used as basic anaerobic (BA) medium as described previously, modified to exclude cysteine hydrochloride, and according to Run 11 in the Plackett-Burman experimental design matrix used in Zhao et al. (2010). pH 5.5 in the medium was obtained by omitting sodium bicarbonate the medium and sparging the flasks with pure CO₂ gas, instead of 80/20 % N₂/CO₂ gas mixture.

All batch experiments to evaluate the influence of the concentration of xylose and other nutrients and to assess ethanol yields were performed in triplicate and with controls where xylose was omitted. 250 ml serum vials containing 100 ml of BA medium with 10% (v/v) inoculums were used throughout the experiments.

### PCR-DGGE and sequence analysis

To confirm the purity of the isolated strain and for preliminary identification, PCR-DGGE technique was used, as described previously. Genomic DNA was extracted from a sample of liquid culture obtained after the final isolation step and purified using a QIAmp DNA Stool Mini Kit (QIAGEN, 15504). Universal primer 1492-r (CGGCTACCTTGTTACGAC) (SEQ ID NO: 1) and bacteria-specific primer 27-f (GTTTGATCCTGGCTCAG) (SEQ ID NO: 2) were used for the first PCR. In addition, the T27_R1 primer (TAAACCACATGCTCCACCGCT) (SEQ ID NO: 3) was used for the 16S sequencing. Primers DGB90 (GCCCGCCGCGCGGCGGGCGGGGCGGGGGCACGGGGGGCCTACGGGA GGCAGCAG) (SEQ ID NO: 4) and DGB99 (ATTACCGCGCTGCTGG) (SEQ ID NO: 5) were used to amplify the V3 region in the second PCR.

The bands corresponding to the products of the second PCR were excised from the DGGE bands and sent for sequencing (MilleGen, Labège, France). The V3 and partial 16S region of the genome were deposited in the GenBank database under the accession number GU176611.

Sequencing results were aligned with the closest relatives and other relevant species using CLUSTALX as previously described. All 16s rRNA sequences were retrieved from Ribosomal Database Project. The distance matrix was calculated by the Jukes-Cantor model and a phylogenetic tree was obtained via the neighbour-joining method.

### Analytical methods

Hydrogen concentration in the headspace was measured in a gas chromatography equipment (MicroLab, Aarhus, Denmark) with a thermal conductivity detector (TCD) and a s-m stainless column packed with Porapak Q (50/80 mesh). Nitrogen was used as the carrier gas.

Xylose and lactic acid (lactate) were determined by high-performance liquid chromatography (HPLC) (Agilent) using a refractive index detector and a Bio-Rad Aminex HPX-87H column operated at 63.5°C; 4 mM H₂SO₄ was used as an eluent at a flow rate of 0.6 ml/min. Detection limits for xylose and lactic acid were 0.001 % (w/v) and 0.0035% (w/v), respectively.

Volatile fatty acids (VFAs) and alcohols (ethanol, butanol) were analyzed using a gas chromatograph (HP 5890 series II) equipped with a flame ionization detector (FID) and an HP FFAP column. The GC-TCD and GC-FID conditions were set according to Liu et al., 2008. Detection limit for VFAs (acetic acid, butyric acid, valeric acid, propionic acid and hexanoic acid) was 0.002% (w/v). Detection limit for ethanol and butanol was 0.0094% (w/v).

### Cell morphology

Cell morphology was examined using a bright field microscope Zeiss Axioskop. Photomicrographs were taken with a camera Leica DFC220 attached to the microscope.

### Substrate utilisation, temperature and pH profiling

The ability of strain DTU01 to utilize other substrates than xylose was determined using BA medium as described before, but with 2 g test substrate I⁻¹ instead of 5 g xylose I⁻¹. Utilization was considered positive when variation in OD₆₀₀ was twice or more than that of the control cultures (containing 1 g yeast extract I⁻¹ as sole carbon source). The temperature and pH profiles, substrate range and tolerance of thiosulphate and sulphite were determined by measuring the variation of optical density (OD) at 600 nm, using a Spectronic 20D+ (Thermo Scientific). For pH range determination experiments, 4 different buffers were used in the media, at a final concentration of 50 mM: Walpole's acetate buffer, pH^{25 °C} range 3.7-5.1; MES sodium salt, pH^{25 °C} range 5.6-6.4; NaHCO₃, pH^{25 °C} range 5.6-7.1; Tris (Trizma base), pH^{25 °C} range 7.5-8.6; and Na₂CO₃, pH^{25 °C} range 8.4-9.5.

### Example 1

### Sequence analysis of DTU01

The V3 sequence is too short to be determinative in terms of taxonomic identification, however analysis of the V3 region preliminarily indicated that microorganism of the present invention was a *Thermoanaerobacter sp*, wherefore the partial 16S and V3 sequence were originally deposited under the accession number GU 176611 as a Thermoanaerobacter sp. Phylogenetic and similarity sequence analysis of the full 16S rDNA sequence of strain DTU01 revealed that it belongs to the class *Clostridia*. The closest relative was *Caldicoprobacter oshimai*, with 98 % similarity. The other closest relatives with validly published names, with 86% similarity were *Thermobrachium celere*, *Thermoanaerobacter ethanolicus*, *Caloranaerobacter azorensis*, and *Catabacterhongkongensis*. All these are thermophilic obligate anaerobic bacteria, with optimum growth temperatures in the range of 65-70 °C.

Despite being a thermophilic anaerobic bacterium, strain DTU01 doesn't cluster with any of the main groups of thermophilic anaerobic bacteria, such as *Thermoanaerobacter*, *Caldicellulosiruptor*, etc. It also forms a separate branch from its closest relative, C. *oshimai* (Figure 1). In figure 1, DTU01 is referred to as "othso".

### Example 2

### Effect of initial xylose concentration

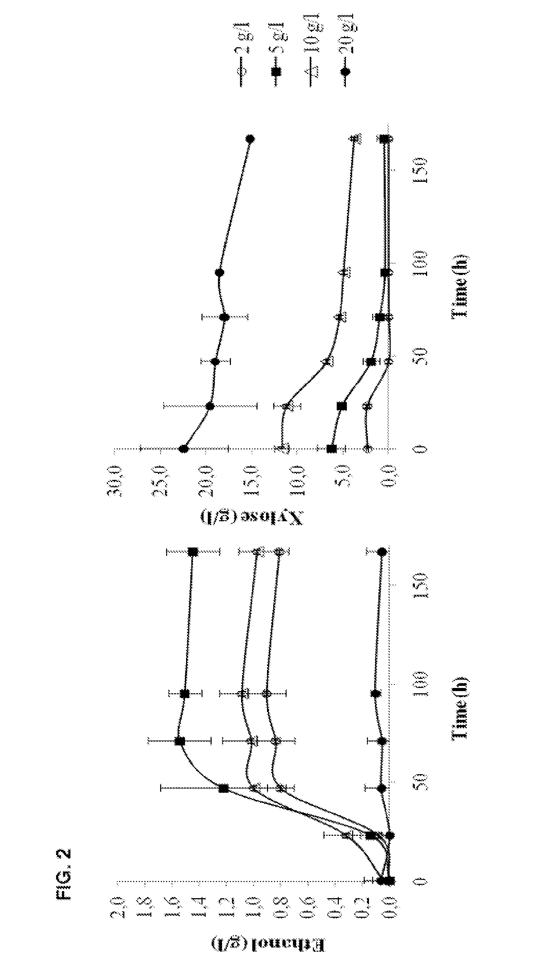

Ethanol production and xylose removal were investigated in batch cultivations at different initial xylose concentrations: 2, 5, 10 and 20 g/I (Figure 2).

For concentrations 2-5 g/l, ethanol production started within the first 24 hours of cultivation and occurred at very close rates. Stationary phase was reached within 71 h. The xylose consumption profiles for these concentrations also show a similar trend, although at 10 g/I xylose was not totally degraded within the same time frame.

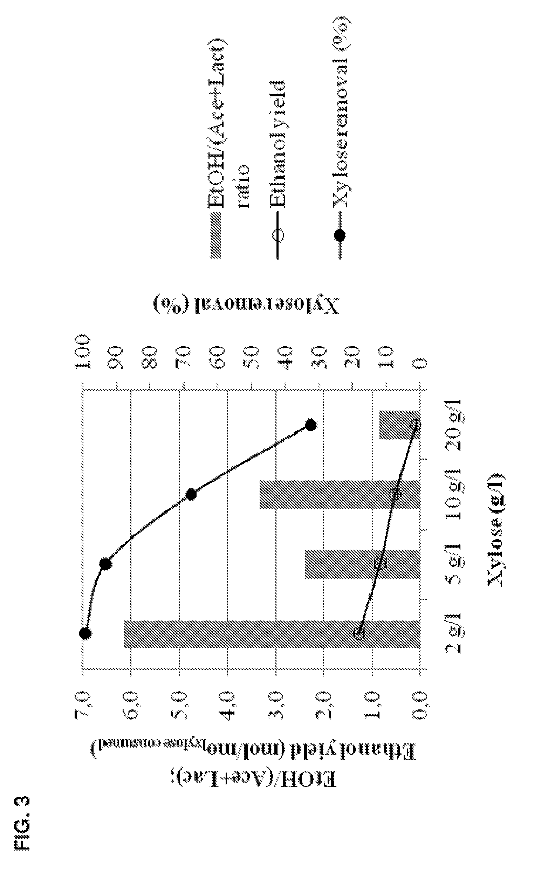

The highest ethanol yield was obtained for an initial concentration of xylose of 2 g/I and was 1.28 molₑₜₕₐₙₒₗ/mol_{xylose} consumed (0.39 gₑₜₕₐₙₒₗ/g_{sugar} consumed), which corresponds to 77% of the theoretical value (1.67 molₑₜₕₐₙₒₗ/mol_{xylose} (0.51 gₑₜₕₐₙₒₗ/g_{sugar consumed})) (Figure 3). To the date of writing, this is the maximum value reported in the literature for thermophilic ethanol production from xylose with a non-engineered pure culture.

Ethanol yields decreased with increasing initial xylose concentration and at 20 g/I, no significant ethanol production was observed. At concentrations above 5 g/l, xylose removal was not complete, which could indicate substrate inhibition.

Another reason for the decreasing ethanol yields could be product inhibition, by accumulation either of fermentation products or of chemical xylose conversion products resulting from caramelization of xylose or Maillard reaction. Indeed, change of the colour to brown with cultivation time was observed in the serum flasks. The darkness of the brown colour increased over time for all the flasks and the intensity of browning was higher in the vials with higher initial xylose concentration.

Nevertheless, it is worth to remember that the inoculum had been cultivated at 2 g/I of xylose during all of the isolation process. Adaptation to higher substrate concentrations has previously been reported for other thermophilic cultures and repeated batch cultivation in higher values could lead to higher yields. Indeed, the isolated organism showed potential to adapt to higher concentrations of xylose, when the yield at 5 g/I increased after several cultivations. Obtaining higher ethanol yields at high substrate concentrations is important, since typical sugar concentrations in, for example, pre-treated lignocellulosic substrates can reach values as high as 51.3 g/I.

The maximum ethanol concentration obtained in the flasks was 1.6 g/I for 5 g/I initial xylose concentration, corresponding to 0.16% w/w. Although this value is not as high as can be obtained with e.g. *Saccharomyces cerevisae*, the value falls within concentrations reported for other ethanologenic thermophilic bacteria. Furthermore, given the volatile properties of ethanol, downstream processing of ethanol produced at high temperatures will not require as high concentrations of ethanol as when ethanol is produced at lower temperatures by mesophilic species. Once the ethanol is in the gas phase, its removal can be done by mild vacuum application, or stripping, as opposed to conventional distillation.

### Example 3

### Effect of peptone and yeast extract concentration

In the enriched mixed culture used as initial inoculum for this isolation process, the highest ethanol yields were achieved in medium supplemented with 2 g/I yeast extract (YE) and 5 g/I peptone. These values are higher than the typical amounts of nutrients and supplements added to culture media. Therefore, the effect of peptone and YE concentration in ethanol production by this isolate were evaluated. The initial concentration of xylose used in these tests was 5 g/I.

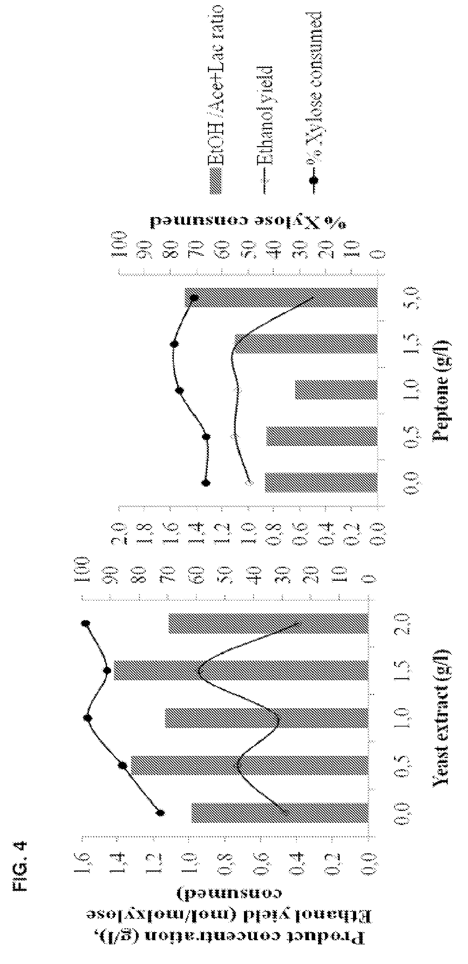

Ethanol to acetate and lactate ratios in Figure 4, show that in almost all the cases, ethanol was the predominant fermentation product. Generally lower yields were achieved in the series of batches were YE was tested. In these batches the peptone concentration used was 5 g/I, therefore these lower yields are in agreement with the also lower yield achieved for this concentration in the peptone series. Lower concentrations of peptone (0-1.5 g/I) did not seem to have an effect on ethanol yield.

Xylose consumption seems to increase until a certain point with both peptone and YE. It has been reported in several studies that supplementing the growth medium with YE generally has beneficial effects to the fermentation process, such as increased hydrogen yields, improved sugar consumption and increased ethanol yields. This is due to the highly rich composition of YE, which includes peptides, amino acids, vitamins and carbohydrates. A similar effect can be expected from the addition of peptone, which contains mostly amino acids.

However, in this case, the highest concentration of peptone (5 g/I) yielded less ethanol and also inverted the growing xylose consumption trend. This can once again be explained by the Maillard reaction, which consists of the reaction of reducing sugars with amino acids, given that in these particular serum flasks in the lower range of peptone added (0-1.5 g/I), the concentration did not seem to have a significant effect in the ethanol yield. In the case of YE, a trend was not observed, but the highest yield was achieved for 1.5 g/I YE added to the medium.

### Example 4

### Other nutrient requirements and pH effect

According to Zhao et al. (2010), 5.5 was the optimal pH value for ethanol production in the enriched mixed culture. However, typical pH values reported for optimal growth of other anaerobic and thermophilic organisms are usually in the range of 6-8, hence the effect of pH was tested. The ability of the new isolate to growth without the addition of vitamins and YE was also tested. The concentration of xylose used in these tests was 5 g/l.

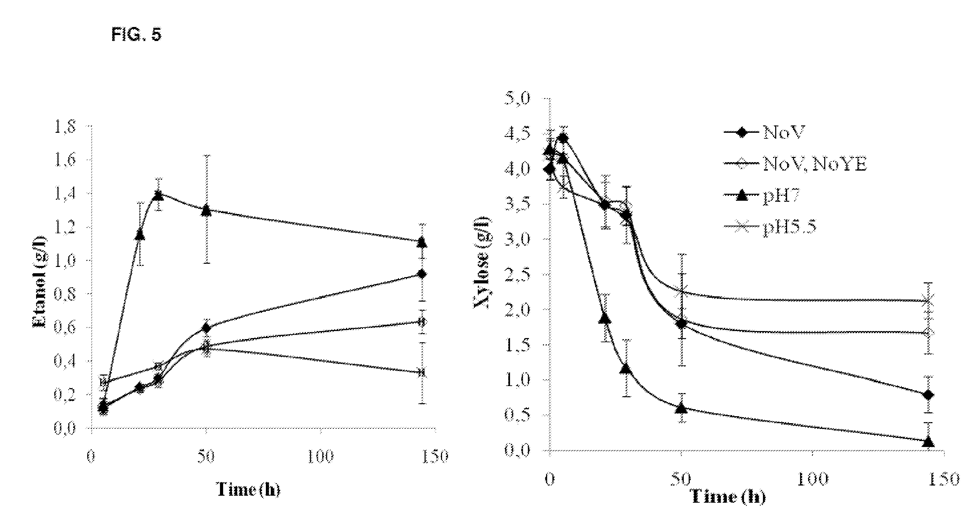

Figure 5 compares ethanol accumulation with xylose consumption over the course of the fermentation. Ethanol production occurred faster and reached higher values at pH 7. This corresponded to the fastest xylose consumption rate and almost full conversion. The decrease in ethanol concentration observed after 60 h of fermentation corresponded to a simultaneous increase both in lactate and acetate concentrations (data not shown). This suggests a shift in the metabolic pathways occurring at lower concentrations of xylose and /or higher concentrations of ethanol.

For the batches at pH 5.5 and where vitamins and YE were omitted from the medium, neither ethanol production rates nor xylose consumption rates varied significantly. The main difference was noticed when the media contained YE but not vitamins: more xylose was consumed when compared to the medium lacking both supplements. This confirms the observation in the previous section that addition of YE to the medium seems to benefit the fermentation towards total sugar utilization.

The fact that the isolate is not significantly affected by the different concentrations of supplements reinforces its value as a promising bioethanol producer. The addition of high quantities of nutritional supplements in an industrial-scale process using second-generation feedstocks as a raw material for ethanol production would be expensive and eradicate the sustainable character of the process.

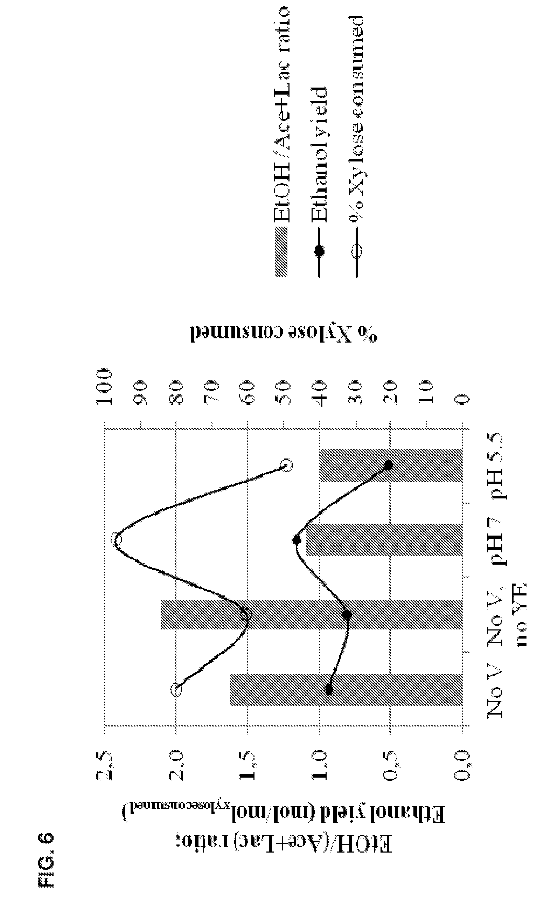

Figure 6 shows that the highest ethanol yield was obtained for pH 7 and was 1.12 molₑₜₕₐₙₒₗ/mol_{xylose consumed} (0,34 gₑₜₕₐₙₒₗ/g_{xylose}) Which accounts for 69% of the theoretical yield. This value is higher than the 0.8 molₑₜₕₐₙₒₗ/mol_{xylose} consumed (0,24 gₑₜₕₐₙₒₗ/g_{xylose}) achieved when the isolate was first cultured with 5 g/l initial xylose concentration. Accordingly, xylose consumption was 97 %, which is also slightly higher than in the beginning for the same conditions - 92 %. This indicates that the isolate has the capacity of adaptation, which is a valuable asset to any potential candidate microorganism to be used in an industrial process.

Although the yields achieved were lower when vitamins and YE were omitted from the culture medium, the isolate was still able to grow and utilize more than 60 % of the xylose added to the medium. In all the situations ethanol was the main fermentation product. Interestingly, the highest ethanol to acetate and lactate ratio was achieved in the non-supplemented medium, with a value of 2.1.

The isolate was thus able to grow and produce significant yields of ethanol in media lacking nutrient sources such as vitamins, peptone or YE. This is a valuable asset for a candidate microorganism for second-generation bioethanol production, meaning this can be achieved without costly addition of supplements to the lignocellulosic biomass.

### Example 5

### Cell morphology

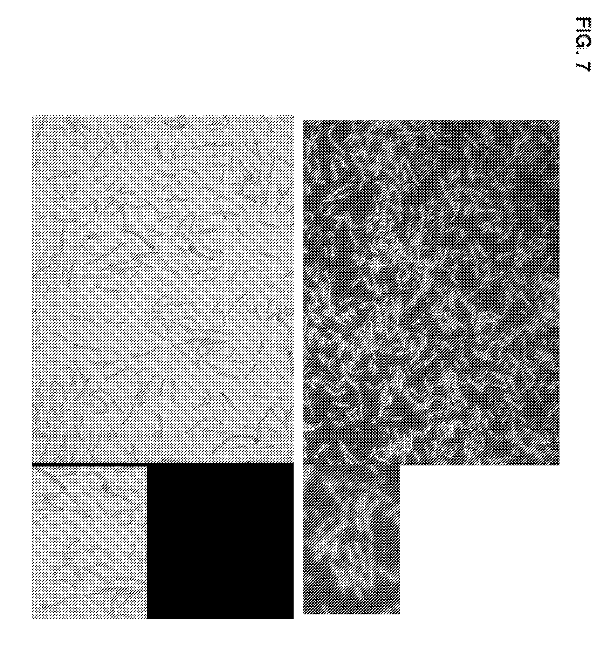

Vegetative cells were rod-shaped, and appeared single, in pairs or in chains (Figure 7). Gram-negative staining was observed both under exponential and stationary phase, although the organism is phylogenetically Gram-positive. Spore staining was performed according to (Schaeffer and Fulton 1933). Sporulation of cells in the stationary phase of growth was observed. Spores were round and terminal (Figure 7).

Sporulating cultures survived autoclavation during 1 h at 120 °C, showing that the spores are heat-resistant.

### Example 6

### Oxygen tolerance

Strain DTU01 was found to be obligately anaerobic, with no detectable growth in flasks supplemented with air and oxygen in the headspace. Oxidation of the medium was indicated by the pink colour of rezasurin.

### Example 7

### Temperature and pH growth range

For pH range determination experiments, four different buffers were used in the media, at a final concentration of 50 mM: Walpole's acetate buffer, pH^{25 °C} range 3.7-5.1; MES sodium salt, pH^{25 °C} range 5.6-6.4; NaHCO₃, pH^{25 °C} range 5.6-7.1; Tris (Trizma base), pH^{25 °C} range 7.5-8.6; and Na₂CO₃, pH^{25 °C} range 8.4-9.5.

The temperature range for growth at pH^{25 °C} was 45-80 °C, with an optimum of 70 °C, and the pH^{25 °C} range for growth at 70 °C was 5.36-8.92. Incubation at higher temperatures and pHs than the growth range resulted in the browning of the medium, caused by caramelization of xylose.

### Example 8

### Physiological characteristics of DTU01 and comparison to closest relatives

The results of the physiological characterization of DTU01 are summarized in Table 1. The data are compared to those of *Caldicoprobacter oshimai, Thermoanaerobacter ethanolicus, Caloranaerobacter azorensis* and *Thermobrachium celere*, i.e. the closest relatives when considering 16S rDNA similarity.

There are several morphologic differences that differentiate strain DTU01 from these organisms. T. *ethanolicus* is the type strain of the genus *Thermoanaerobacter*, which comprises a large number of species. In contrast, T. *celere*, C. *azorensis* and C. *oshimai* are the sole representatives of their respective genera. Despite having the same optimum growth temperature as strain DTU01, the pH growth range is wider in T. *ethanolicus*.

DTU01 stains gram-negative in all phases of growth, and it is able to degrade starch, while C. *oshimai* lacks this ability.

DTU01 exhibits a large evolutionary distance from other anaerobic/thermophilic/ethanol producers of the clostridium subphylum.

DTU01 did not have the absolute growth requirement for yeast extract as described previously for genus *Thermoanaerobacter*. This supplement could be replaced by the use of a vitamin solution.

DTU01 is the only microorganism among its closest relatives to have been isolated from household waste. Others have been isolated from animal faeces, hot springs and other thermophilic environments.

Caldicoprobacter strain DTU01 can grow at 55-80 °C, pH 5.5-8.5, but the maximum yield so far was achieved at 70 °C and pH 7. It can ferment most sugars, cf. Table 1. It is also able to ferment sugar mixtures of glucose and xylose.

Growth in rapeseed straw hydrolysate was tested and positive at 5% w/v. Higher values of dry matter have not yet been tested but it is likely that DTU01 can grow at higher dry matter concentrations.

Immobilization of the strain is being tested in several supports - activated carbon and rapeseed straw - and it is showing positive results when the immobilized cells are being used in continuous reactors. Immobilization is important for industrial purposes.

The main fermentation products of DTU01 when using 5 g xylose I⁻¹ as a carbon source were ethanol, hydrogen, lactate and acetate. Typical yields were respectively 0.95, 0.15, 0.19 and 0.30 mol product per mol xylose degraded corresponding to 0.29, 0.002, 0.076 and 0.18 g_{product}/g_{xyolse consumed}.

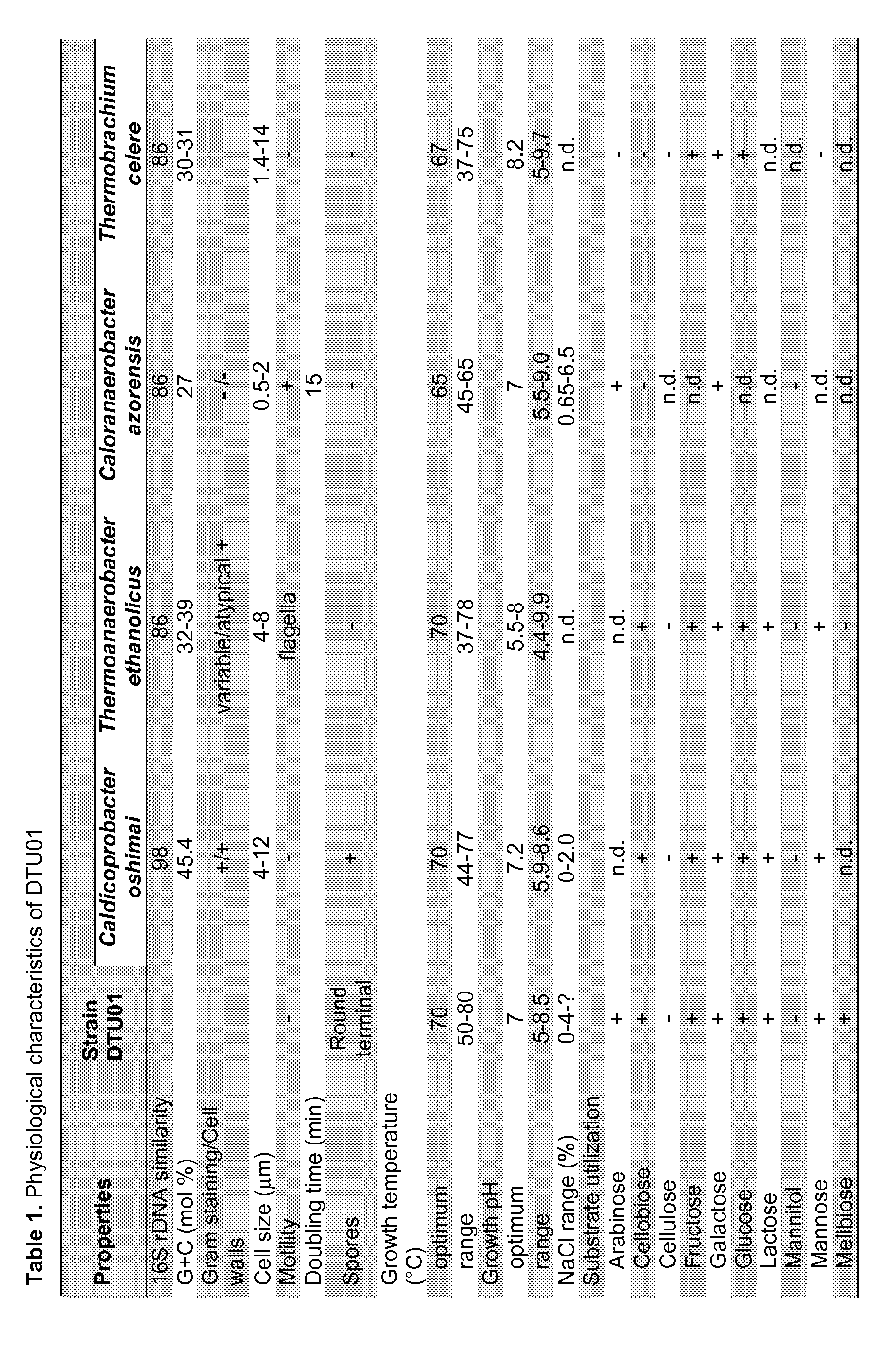

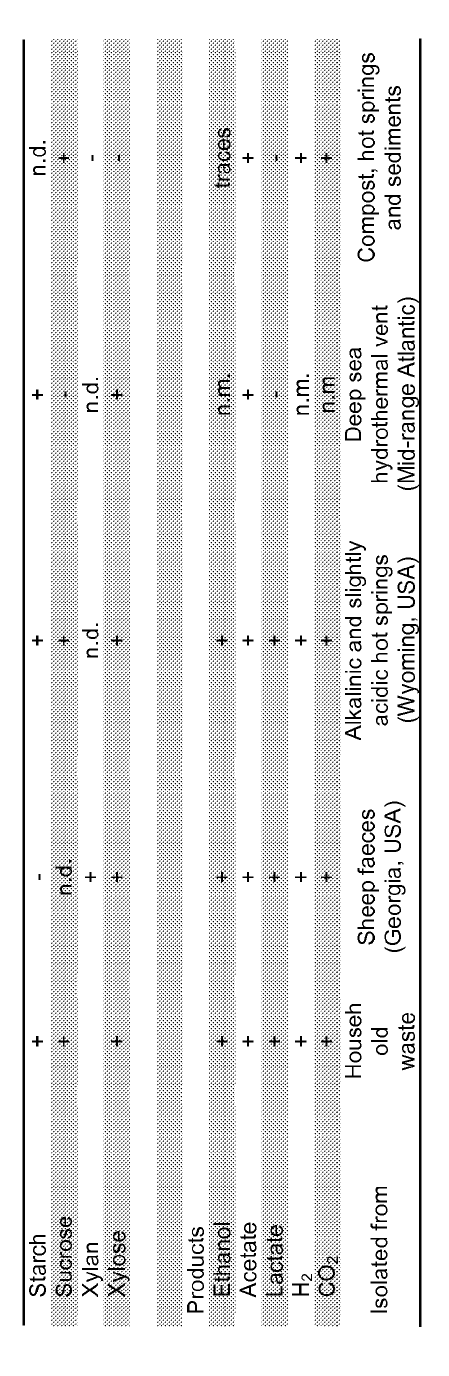

### Example 9

### Improving ethanol tolerance and other growth aspects

Strain DTU01 can grow unaffected in up to 2% v/v ethanol, and it can still grow, albeit at slower rates, at 4% v/v ethanol in the medium. At 5% no growth was reported.

Adaptation to higher concentrations of ethanol can be achieved by repeated batch cultivation technique, which consists of transferring the same culture to increasing concentrations of ethanol after an acclimatization period in each concentration. This is also a suitable method for adaptation to other inhibitors and also to higher concentrations of substrate.

In addition, or alternatively, the tolerance to ethanol and other inhibitors can be improved by genetic modification of the organism. Methods that can be used to genetically modify bacteria include, but are not limited to random mutagenesis, gene insertion and gene knock-out (by inactivation, deletion or mutation).

Tolerance of DTU01 to ethanol and other inhibitors will be improved by both cultivation techniques and by genetic modification of the organism to obtain DTU01 mutants with improved growth characteristics.

### Example 10

### Mutants of DTU01

To obtain mutants or derivatives of strain DTU01 with improved characteristics, such as e.g. strains exhibiting higher fermentation product yields, different fermentation profiles (i.e. to improve the yield of a specific fermentation product) and/or broader substrate utilisation profiles, the DTU01 strain is to be genetically modified.

The genetic modification comprises insertion, deletion or substantial inactivation of one or more genes. Genes may be inserted, deleted or substantially inactivated using suitable gene manipulation tools and genetic engineering procedures which are well known in the art, e.g. gene cloning systems, homologous recombination and techniques described in Sambrook & Russell "Molecular Cloning: A Laboratory Manual" (Third Edition), Cold Spring Harbor Laboratory Press.

The microorganism may also be genetically modified by subjecting the microorganism of the present invention to ionizing radiation or chemical mutagens known to the skilled person.

### References

Cripps, R., Eley, K., Leak, D., Rudd, B., Taylor, M., Todd, M., et al. (2009). Metabolic engineering of Geobacillus thermoglucosidasius for high yield ethanol production. Metabolic engineering, 11 (6), 398-408.

Georgieva, T. I., & Ahring, B. K. (2007). Evaluation of continuous ethanol fermentation of dilute-acid corn stover hydrolysate using thermophilic anaerobic bacterium Thermoanaerobacter BG1 L1. Applied microbiology and biotechnology, 77(1), 61-8.

Shaw, A., Podkaminer, K., Desai, S., Bardsley, J., Rogers, S., Thorne, P., et al. (2009). Metabolic engineering of a thermophilic bacterium to produce ethanol at high yield. International Sugar Journal, 111(1323), 164-171.

Zhao, C., Karakashev, D., Lu, W., Wang, H., & Angelidaki, I. (2010). Xylose fermentation to biofuels (hydrogen and ethanol) by extreme thermophilic (70°C) mixed culture. International Journal of Hydrogen Energy, 35(8), 3415-3422.

Zhao, C., O-Thong, S., Karakashev, D., Angelidaki, I., Lu, W., Wang, H., et al. (2009). High yield simultaneous hydrogen and ethanol production under extreme-thermophilic (70°C) mixed culture environment. International Journal of Hydrogen Energy, 34(14), 5657-5665.

Functional analysis of Bacterial genes: A practical Manual, edited by W. Schumann, S. D. Ehrlich & N. Ogasawara, 2001.

Sambrook & Russell "Molecular Cloning: A Laboratory Manual" (Third Edition), Cold Spring Harbor Laboratory Press

## Claims

1. A microorganism selected from the strain deposited as DSMZ Accession number 24726 and mutants thereof.

2. The microorganism according to claim 1, wherein said microorganism produces ethanol as the main fermentation product.

3. The microorganism according to any of the preceding claims, wherein said microorganism is capable of fermenting pentoses.

4. The microorganism according to any of the preceding claims, wherein said microorganism is capable of co-fermenting hexoses and pentoses.

5. The microorganism according to any of the preceding claims, wherein said microorganism is capable of growing at or above 70°C.

6. The microorganism according to any of the preceding claims, wherein said microorganism is capable of growing in a medium comprising a hydrolysed lignocellulosic biomass material having a dry-matter content of at least 5% w/v, including 10% w/v, such as at least 15% w/v, including at least 20% w/v, such as at least 25% w/v.

7. The microorganism according to claim 6, wherein the hydrolysed lignocellulosic biomass material is selected from the group consisting of garden refuse, comminuted wood, straw, hay, fruit hulls and seed hulls.

8. A method of producing a fermentation product comprising culturing the microorganism according to any of the preceding claims under suitable conditions.

9. The method according to claim 8, wherein the culturing is performed under anaerobic conditions.

10. The method according to any of claims 8-9, wherein the culturing is performed at temperatures in the range of about 40-95°C, such as the range of about 50-90°C, including the range of about 60-85°C, such as in the range of about 65-75°C.

11. The method according to any of claims 8-10, wherein the culturing is performed as a batch fermentation process or a continuous fermentation process.

12. The method according to any of claims 8-11, wherein the fermentation product is selected from the group consisting of ethanol, acetate, lactate, hydrogen, propanol and propionate.

13. The method according to claim 12, wherein the fermentation product is ethanol.

14. A method for producing a mutant of the microorganism deposited as DSMZ accession number 24726 comprising subjecting the microorganism to ionizing radiation or chemical mutagens.


SEQUENCE LISTING

<110>  Technical University of Denmark

<120>  Bacteria for second generation bio-ethanol production

<130>  P2491EP00

<160>  7

<170>  PatentIn version 3.5

<210>  1
<211>  18
<212>  DNA
<213>  Artificial Sequence

<220>
<223>  PCR primer

<400>  1
cggctacctt gttacgac                                                    18

<210>  2
<211>  17
<212>  DNA
<213>  Artificial Sequence

<220>
<223>  PCR primer

<400>  2
gtttgatcct ggctcag                                                     17

<210>  3
<211>  21
<212>  DNA
<213>  Artificial Sequence

<220>
<223>  PCR primer

<400>  3
taaaccacat gctccaccgc t                                                21

<210>  4
<211>  54
<212>  DNA
<213>  Artificial Sequence

<220>
<223>  PCR primer

<400>  4
gcccgccgcg cggcgggcgg ggcgggggca cggggggcct acgggaggca gcag             54

<210>  5
<211>  16
<212>  DNA
<213>  Artificial Sequence

<220>
<223>  PCR primer

<400>  5
attaccgcgc tgctgg                                                      16

<210>  6
<211>  1253
<212>  DNA
<213>  DTU01 deposited under DSMZ Accession number 24726


<220>
<221>  misc_feature
<222>  (7)..(7)
<223>  n is a, c, g, or t

<220>
<221>  misc_feature
<222>  (1251)..(1251)
<223>  n is a, c, g, or t

<400>  6
ggccttnaag cgtcggtaca agacgggctc gcgtcccatt agctagttgg tgaggtaacg      60

gctcaccaag gcgacgatgg gtagccggcc tgagagggtg tacggccaca ctgggactga     120

gacacggccc agactcctac gggaggcagc agtggggaat attgcgcaat gggggaaacc     180

ctgacgcagc gacgccgcgt gagcgaggaa ggccttcggg ttgtaaagct ctgtccccgg     240

ggaagaagga atgacggtac ctggggagga agccccggct aactacgtgc cagcagccgc     300

ggtaatacgt agggggcgag cgttgtccgg agttactggg cgtaaagggt gcgtagggcg     360

gcccattaag tcaggtgtga aaaaccctgg ctcaaccggg gtattgcact tgaaactggt     420

gggcttgagt gcaggagagg gaagtggaat tcctggtgta gcggtgaaat gcgtagatat     480

caggaggaac accagtggcg aaggcggctt cctggcctgt aactgacgct gaggcacgaa     540

agcgtgggga gcaaacagga ttagataccc tggtagtcca cgctgtaaac gatggatact     600

aggtgtaggg ggcgatttag ccttctgtgc cgaagttaac gcaataagta tcccgcctgg     660

ggagtacggc cgcaaggttg aaactcaaag gaattgacgg gggcccgcac aagcggtgga     720

gcatgtggtt taattcgaag caacgcgaag aaccttacca gggcttgaca tgcccctgaa     780

gtatctggaa acaggtacgg cctatgcctt cgggtatagg acaggggaca caggtggtgc     840

atggttgtcg tcagctcgtg tcgtgaatgt tgggttaagt cccgcaacga gcgcaaccct     900

tatccctagt tgccagcgcg ttatggcggg gactctaggg agactgccgg ggagaactcg     960

gaggaaggtg gggatgacgt caaatcatca tgccccttat gccctgggct acacacgtgc    1020

tacaatggcc gggtacagag ggaggcgaag cggtaacgcg gagcgaatcc caaaaaaccg    1080

gtctcagttc ggattgtggg ctgcaactcg cccacatgaa gtcggaatcg ctagtaatcg    1140

cggatcagca tgccgcggtg aatacgttcc cgggccttgt acacaccgcc cgtcacacca    1200

tgggagttgg gggcacccga agccggtggc ctaacccgga aagggaggga nct           1253


<210>  7
<211>  143
<212>  DNA
<213>  DTU01 deposited under DSMZ Accession number 24726

<400>  7
aatcttgggc aatgggcgga agcctgaccc agcgacgccg cgtgggggag gaaggccttc      60

gggttgtaaa ccccgttagt gtgggaagaa gggatgacgg taccacacga aagccccggc 120
taactacgtg ccagcagcgc ggt 143